(19) Europäisches Patentamt
European Patent Office
Office européen des brevets

(11) Veröffentlichungsnummer: **0 046 870**
**B1**

(12) **EUROPÄISCHE PATENTSCHRIFT**

(45) Veröffentlichungstag der Patentschrift: **14.03.84**

(51) Int. Cl.³: **C 07 C 19/07**, C 07 C 17/18, B 01 J 31/28

(21) Anmeldenummer: **81105764.5**

(22) Anmeldetag: **22.07.81**

(54) **Verfahren zur Herstellung wasserfreier Alkyljodide.**

(30) Priorität: **01.09.80 DE 3032853**

(43) Veröffentlichungstag der Anmeldung: **10.03.82 Patentblatt 82/10**

(45) Bekanntmachung des Hinweises auf die Patenterteilung: **14.03.84 Patentblatt 84/11**

(84) Benannte Vertragsstaaten: **AT BE CH DE FR GB IT LI NL**

(56) Entgegenhaltungen:
**DE - A - 2 211 231**
**DE - A - 2 610 036**

(73) Patentinhaber: **HOECHST AKTIENGESELLSCHAFT, Postfach 80 03 20, D-6230 Frankfurt am Main 80 (DE)**

(72) Erfinder: **Kübbeler, Hans Klaus, Dr., Bünnagelring 31, D-5357 Swisttal 8 (DE)**
Erfinder: **Erpenbach, Heinz, Dr., Oberbuschweg 22, D-5000 Köln 50 (DE)**
Erfinder: **Gehrmann, Klaus, Dr., Geschwister-Scholl-Strasse 32, D-5042 Erftstadt (DE)**
Erfinder: **Schmitz, Klaus, Rodderweg 46, D-5042 Erftstadt 15 (DE)**

EP 0 046 870 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung wasserfreier Alkyljodide

Wasserfreie Alkyljodide werden in der präparativen organischen Chemie häufig für Alkylierungen benötigt. In letzter Zeit eröffnen sich für wasserfreie Alkyljodide, insbesondere Methyljodid, auch großtechnische Einsatzmöglichkeiten, z. B. ist gemäß dem in der DE-OS 2 610 036 beschriebenen Verfahren zur Herstellung von Essigsäureanhydrid aus Methylacetat ein Einsatz von wasserfreiem $CH_3I$ unerläßlich.

Bekannte Verfahren zur Herstellung von Alkyljodiden laufen entweder in wäßrigem Milieu ab oder bei ihrer Herstellung entsteht Wasser. So beschreibt die US-PS 3 784 518 die Herstellung von Alkyljodiden wie $CH_3I$ aus Alkanolen wie $CH_3OH$ mit Jod und Wasserstoff an Rh-Kontakten in der Gasphase:

$$2\ CH_3OH + H_2 + I_2 \rightarrow 2\ CH_3I + 2\ H_2O$$

Weiterhin beschreibt die DE—OS 2 211 231 ein Verfahren zur Herstellung von Alkyljodiden, insbesondere $CH_3I$, in wäßriger Lösung durch Umsetzung von $CH_3OH$ mit Jod und CO an Rhodium- bzw. Iridiumkontakten nach folgender Gesamtgleichung:

$$2\ CH_3OH + I_2 + CO \rightarrow 2\ CH_3I + CO_2 + H_2O$$

Diese Verfahren haben den Nachteil, daß vor einem unter wasserfreien Bedingungen erforderlichen Einsatz des Alkyljodids eine sorgfältige Abtrennung des Alkyljodids vom Wasser erfolgen muß. Hieraus ergibt sich die Notwendigkeit einer Herstellung von beispielsweise $CH_3I$ in wasserfreiem Milieu und ohne Anfall von Reaktionswasser.

Die Erfindung betrifft nun ein Verfahren zur Herstellung wasserfreier Alkyljodide, welches dadurch gekennzeichnet ist, daß man Carbonsäurealkylester der Formel $R^1COOR^2$, worin $R^1$ Wasserstoff oder einen Alkyl- oder Arylrest mit 1 bis 8 C-Atomen und $R^2$ einen Alkylrest mit 1 bis 4 C-Atomen bedeuten, mit Jod und Wasserstoff und gegebenenfalls Kohlenmonoxid in Gegenwart von Verbindungen der Edelmetalle Rhodium, Iridium, Palladium oder Ruthenium als Katalysator sowie einer heterocyclischen aromatischen Verbindung, in der mindestens ein Heteroatom ein quaternäres Stickstoffatom ist, oder einer quaternären Organophosphorverbindung als Promotor sowie gegebenenfalls in Gegenwart von Carbonsäure mit 1 bis 8 C-Atomen und/oder deren Anhydriden unter praktisch wasserfreien Bedingungen bei Temperaturen von 350 bis 420 K und Gesamtdrücken bis zu 30 bar umsetzt.

Das Verfahren läuft nach folgender Gesamtgleichung ab:

$$I_2 + 2\ R^1COOR^2 + H_2 \rightarrow 2\ R^1COOH + 2\ R^2I$$

Weiterhin kann die Erfindung bevorzugt und wahlweise dadurch gekennzeichnet sein, daß man

a) die heterocyclischen Verbindungen oder Organophosphorverbindungen in Form ihrer Addukte mit Essigsäure oder Methyljodid einsetzt,
b) das Katalysatorsystem Edelmetallverbindung/Promotor/Carbonsäure(-anhydrid) im Molverhältnis 1 : (5—500) : (0—700) einsetzt,
c) Wasserstoff/Kohlenstoffmonoxid-Gemische mit bis zu 35 Volum-% Kohlenmonoxid einsetzt.

Als heterocyclische aromatische Verbindungen und als quaternäre Organophosphorverbindungen kommen erfindungsgemäß beispielsweise infrage:

1. N-Methylpyridiniumjodid, N,N-Dimethylimidazoliumjodid, N-Methyl-3-picoliniumjodid, N-Methyl-2,4-lutidiniumjodid, N-Methyl-3,4-lutidiniumjodid, N-Methyl-chinoliniumjodid;
2. Pyridiniumacetat, N-Methylimidazoliumacetat, 3-Picoliniumacetat, 2,4-Lutidiniumacetat, 3,4-Lutidiniumacetat;
3. Tributyl-methyl-phosphoniumjodid, Trioctyl-methyl-phosphoniumjodid, Trilauryl-methyl-phosphoniumjodid, Triphenyl-methyl-phosphoniumjodid.

Das bei dem erfindungsgemäßen Verfahren anfallende wasserfreie Alkyljodid, z. B. $CH_3I$, kann durch Destillation aus dem Reaktionsprodukt ohne Schwierigkeiten gewonnen werden. Die verbleibende Reaktionslösung wird nach Zusatz der dem Umsatz entsprechenden Mengen Jod und Carbonsäurealkylester erneut in den Reaktionszyklus eingesetzt. Andererseits kann die gewonnene Reaktionslösung ohne Isolierung des nach dem Verfahren der Erfindung hergestellten $CH_3I$ direkt im Verfahren zur Herstellung von Essigsäureanhydrid aus Methylacetat und Kohlenmonoxid gemäß DE-OS 2 450 965 und DE-OS 2 836 084 eingesetzt werden. Umgekehrt läßt sich die für den Acetanhydridprozeß verwendete Katalysatorlösung nach Zusatz von Jod und Methylacetat zur Herstellung von $CH_3I$ gemäß der Erfindung benutzen.

Beim Verfahren der Erfindung ist ein intensiver Kontakt des Wasserstoffs und gegebenenfalls des

Kohlenmonoxids mit der Reaktionslösung erforderlich, was durch Eindüsen des Gases bzw. Gasgemisches in die Reaktionslösung und gegebenenfalls durch Rühren der Reaktionslösung erreicht wird.

Der Gesamtdruck soll 30 bar nicht überschreiten. Der Wasserstoffpartialdruck kann bis zu 20 bar betragen. CO-Partialdrücke oberhalb 10 bar verursachen keine Verbesserung im gewünschten Reaktionsablauf. Die Edelmetallmenge kann in Grenzen von 0,0001—0,01 Mol je Mol Carbonsäurealkylester schwanken.

Beispiel 1

In einem Druckgefäß aus Edelstahl werden 1,2 g $[Rh(Co)_2Cl]_2$ in 250 g Methylacetat und 50 g Essigsäure mit 60 g N,N-Dimethylimidazoliumjodid versetzt. Nach einer Zugabe von 50 g Jod werden 2 bar CO und 4 bar Wasserstoff aufgedrückt. Anschließend wird 58 min unter Rühren auf 373 K erhitzt. Im Reaktionsgemisch werden gaschromatographisch 55,7 g $CH_3I$ nachgewiesen. Die Ausbeute errechnet sich zu 99,6% der Theorie, bezogen auf eingesetztes Jod.

Beispiel 2

In einem Druckgefäß werden 5 g $PdCl_2[P(C_6H_5)_3]_2$ in 250 g Methylacetat und 50 g Essigsäure mit 100 g Methyltriphenylphosphoniumjodid vorgelegt. Nach einer Zugabe von 50 g Jod werden 4 bar CO und 8 bar Wasserstoff aufgedrückt. Man erhitzt 78 min unter Rühren auf 383 K und erhält 55,6 g $CH_3I$.

Beispiel 3

In einem Druckgefäß werden 1,9 g $[IrCl(CO)_3]_2$ in 250 g Methylacetat und 50 g Essigsäure mit 65 g N-Methyl-3-picoliniumjodid vorgelegt. Nach einer Zugabe von 50 g Jod werden 2 bar CO und 4 bar Wasserstoff aufgedrückt. Zweistündiges Erhitzen unter Rühren auf 373 K liefert 55,7 g $CH_3I$.

Beispiel 4

In einem Druckgefäß werden 1,6 g $[RuCl_2(CO)_3]_2$ in 250 g Methylacetat und 50 g Essigsäure mit 70 g N,N-Dimethylimidazoliumjodid versetzt. Im Anschluß an eine Zugabe von 50 g Jod werden 4 bar CO und 8 bar Wasserstoff aufgedrückt. Nach 72minütigem Erhitzen auf 393 K unter Rühren können 55,6 g $CH_3I$ im Reaktionsgemisch nachgewiesen werden.

Beispiel 5

In einem Druckgefäß werden 2 g $[Rh(CO)_2Cl]_2$ in 250 g Methylacetat und 60 g Essigsäureanhydrid mit 70 g N,N-Dimethylimidazoliumjodid versetzt. Nach Zugabe von 100 g Jod werden 2 bar CO und 4 bar Wasserstoff aufgedrückt und 83 min bei 383 K unter Rühren bei etwa 10 bar und Nachströmen von Wasserstoff umgesetzt. Analytisch werden im Reaktionsgemisch 110,3 g $CH_3I$ ermittelt.

Beispiel 6

In einem Druckgefäß werden 1,8 g $[Rh(CO)_2Cl]_2$ in 250 g Methylacetat und 60 g Essigsäureanhydrid mit 50 g N-Methyl-3-picoliniumjodid versetzt. Nach Zugabe von 100 g Jod wird 6 bar Wasserstoff aufgedrückt und auf 383 K erhitzt. Nach einer Reaktionszeit von 88 min unter Nachströmen von Wasserstoff über eine Gasverteilungsfritte bei etwa 10 bar werden 110,7 g $CH_3I$ ermittelt.

Beispiel 7

50 g Katalysatorsumpf aus der Acetanhydridherstellung, im wesentlichen bestehend aus N,N-Dimethylimidazoliumjodid, das 0,15 g Rh enthält, wird in einem Druckgefäß mit 250 g Methylacetat versetzt. Nach Zugabe von 100 g Jod wird 5 bar Wasserstoff zugesetzt und auf 393 K erhitzt. Die Reaktion ist unter Rühren und Wasserstoffnachströmen bei etwa 9 bar in 52 min beendet. Es lassen sich 111 g $CH_3I$ nachweisen.

Beispiel 8

In einem Druckgefäß werden 1,5 g $[Rh(CO)_2Cl]_2$ in 300 g Benzoesäuremethylester und 20 g Benzoesäure mit 60 g N,N-Dimethylimidazoliumjodid versetzt. Nach einer Zugabe von 50 g Jod werden 2 bar CO und 4 bar Wasserstoff aufgedrückt. Anschließend wird 40 min unter Rühren auf 398 K erhitzt. Im Reaktionsgemisch werden 55,3 g $CH_3I$ ermittelt.

Beispiel 9

In einem Druckgefäß werden 1,2 g $[Rh(CO)_2Cl]_2$ in 250 g Äthylpropionat und 60 g Propionsäure mit 60 g N-Methyl-3-picoliniumjodid und 50 g Jod versetzt. Nach Aufdrücken von 2 bar CO und 8 bar Wasserstoff wird 37 min unter Rühren auf 393 K erhitzt. Im Reaktionsgemisch werden 60,5 g $C_2H_5I$ nachgewiesen.

Beispiel 10

In einem Druckgefäß werden 0,1 g $[Rh(CO)_2Cl]_2$ in 250 g Methylacetat und 20 g Essigsäure mit 75 g Methyl-tributyl-phosphoniumjodid versetzt. Nach einer Zugabe von 25 g Jod werden 4 bar CO und 8 bar Wasserstoff aufgedrückt. Anschließend wird 118 min unter Rühren auf 393 K erhitzt. Im Reaktionsgemisch werden 27,8 g $CH_3I$ nachgewiesen.

Beispiel 11

In einem Druckgefäß werden 5,9 g $[Rh(CO)_2Cl]_2$ in 250 g Methylacetat und 60 g N,N-Dimethylimidazoliumjodid eingesetzt. Nach Zugabe von 60 g Jod werden 2 bar CO und 4 bar Wasserstoff aufgedrückt. Nach 28minütigem Erhitzen auf 383 K unter Rühren und Nachströmen von Wasserstoff bei etwa 10 bar werden 67 g $CH_3I$ im Reaktionsprodukt nachgewiesen.

Beispiel 12

In einem Hastelloy-Druckgefäß, ausgestattet mit Rührer, werden 6 g $[Rh(CO)_2Cl]_2$ in 1550 g Methylacetat und 500 g Essigsäure mit 250 g N,N-Dimethylimidazoliumjodid versetzt. Nach einer Zugabe von 2500 g Jod werden 2 bar CO und 4 bar Wasserstoff aufgedrückt. Dann wird auf 383 K erhitzt, wobei man bei etwa 10 bar Gesamtdruck Wasserstoff nachströmen läßt. Sobald die Analysen des Gasraumes ein stärkeres Auftreten von Methan anzeigen, ist die Reaktion beendet und das entstandene Methyljodid wird destillativ gewonnen. Es fallen 2791 g $CH_3I$ an, was einer quantitativen Ausbeute entspricht. Der Katalysatorsumpf wird entsprechend dem Umsatz mit neuen Ausgangsstoffen (Methylacetat und Jod) mehrfach zur Herstellung weiteren Methyljodids unter gleichen Bedingungen ohne Aktivitätsverlust eingesetzt.

**Patentansprüche**

1. Verfahren zur Herstellung wasserfreier Alkyljodide, dadurch gekennzeichnet, daß man Carbonsäurealkylester der Formel $R^1COOR^2$, worin $R^1$ Wasserstoff oder einen Alkyl- oder Arylrest mit 1 bis 8 C-Atomen und $R^2$ einen Alkylrest mit 1 bis 4 C-Atomen bedeuten, mit Jod und Wasserstoff und gegebenenfalls Kohlenmonoxid in Gegenwart von Verbindungen der Edelmetalle Rhodium, Iridium, Palladium oder Ruthenium als Katalysator sowie einer heterocyclischen aromatischen Verbindung, in der mindestens ein Heteroatom ein quaternäres Stickstoffatom ist, oder einer quaternären Organophosphorverbindung als Promotor sowie gegebenenfalls in Gegenwart von Carbonsäuren mit 1 bis 8 C-Atomen und/oder deren Anhydriden unter praktisch wasserfreien Bedingungen bei Temperaturen von 350 bis 420 K und Gesamtdrücken bis zu 30 bar umsetzt.

2. Verfahren nach Anspruch 1, dadurch gekennzeichnet, daß man die heterocyclischen Verbindungen oder Organophosphorverbindungen in Form ihrer Addukte mit Essigsäure oder Methyljodid einsetzt.

3. Verfahren nach einem der vorhergehenden Ansprüche, dadurch gekennzeichnet, daß man das Katalysatorsystem Edelmetallverbindung/Promotor/Carbonsäure(-anhydrid) im Molverhältnis 1 : (5—500) : (0—700) einsetzt.

4. Verfahren nach einem der vorhergehenden Ansprüche, dadurch gekennzeichnet, daß man Wasserstoff/Kohlenstoffmonoxid-Gemische mit bis zu 35 Volum% Kohlenmonoxid einsetzt.

**Claims**

1. Process for making anhydrous alkyl iodides, which comprises: reacting under practically anhydrous conditions at temperatures of 350 to 420 K and under a total pressure of up to 30 bars, carboxylic acid alkyl esters of the formula $R^1COOR^2$, in which $R^1$ stands for an alkyl or aryl radical having from 1 to 8 carbon atoms and $R^2$ stands for an alkyl group having from 1 to 4 carbon atoms, with iodine and hydrogen and optionally carbon monoxide in the presence of compounds of the noble metals rhodium, iridium, palladium or ruthenium as catalyst, and of a heterocyclic aromatic compound, in which at least

one hetero atom is a quaternary nitrogen atom, or of a quaternary organo-phosphorus compound as a promoter, and optionally also in the presence of carboxylic acids having from 1 to 8 carbon atoms and/or their anhydrides.

2. Process as claimed in claim 1, wherein the heterocyclic compounds or organophosphorus compounds are used in the form of their addition products with acetic acid or methyl iodide.

3. Process as claimed in any of the preceding claims, wherein the catalyst system comprised of noble metal compound/promoter/carboxylic acid (-anhydride) is used in a molar ratio of 1 : (5—500) : (0—700).

4. Process as claimed in any of the preceding claims, wherein hydrogen/carbon monoxide mixtures containing up to 35 volume % of carbon monoxide are used.

**Revendications**

1. Procédé de préparation d'iodures d'alkyles anhydres, caractérisé en ce que l'on fait réagir, dans des conditions pratiquement anhydres, à des températures de 350—420 K et sous des pressions totales allant jusqu'à 30 bars, des carboxylates d'alkyle de formule $R^1COOR^2$, dans laquelle $R^1$ représente un radical alkyle ou aryle en $C_1—C_8$ et $R^2$ représente un radical alkyle en $C_1—C_4$, avec l'iode et l'hydrogène et, éventuellement, le monoxyde de carbone en présence de composés des métaux nobles rhodium, iridium, palladium ou ruthénium comme catalyseurs ainsi que d'un composé aromatique hétérocyclique, dans lequel au moins un hétéroatome est un atome d'azote quaternaire, ou d'un composé organophosphoré quaternaire comme promoteur ainsi éventuellement que d'acides carboxyliques en $C_1—C_8$ et/ou de leurs anhydrides.

2. Procédé selon la revendication 1, caractérisé en ce que l'on utilise les composés hétérocycliques ou composés organo-phosphorés sous forme de leurs produits d'addition sur l'acide acétique ou l'iodure de méthyle.

3. Procédé selon l'une des revendications précédentes, caractérisé en ce que l'on utilise le système catalytique composé de métal noble/promoteur/(anhydride d')acide carboxylique dans le rapport molaire de 1 : (5—500) : (0—700).

4. Procédé selon l'une des revendications précédentes, caractérisé en ce que l'on utilise des mélanges monoxyde de carbone/hydrogène contenant jusqu'à 35% en volume de monoxyde de carbone.